# FASCICULE DE BREVET EUROPEEN

(11) **EP 3 592 869 B1**
(45) Date de publication et mention de la délivrance du brevet: **28.04.2021**
(21) Numéro de dépôt: 18714717.8
(22) Date de dépôt: 09.03.2018
(51) Int. Cl.: A61K 35/74, C12N 1/20, C12R 1/01, C12R 1/145, A61K 35/00, A23P 10/30, A61P 3/00, A61P 3/04

(54) **BACTERIES DE LA FAMILLE DES CHRISTENSENELLACÉES POUR LEUR UTILISATION COMME MEDICAMENT EN PARTICULIER POUR LUTTER CONTRE L'OBESITE, LES MALADIES CARDIOMETABOLIQUES ET LES MALADIES INFLAMMATOIRES DE L'INTESTIN**
BAKTERIEN DER CHRISTENSENELLACEAE FAMILIE ZUR VERWENDUNG ALS ARZNEIMITTEL, INSBESONDERE ZUR BEHANDLUNG VON FETTLEIBIGKEIT, KARDIOMETABOLISCHEN ERKRANKUNGEN UND ENTZÜNDLICHEN DARMERKRANKUNGEN
BACTERIA OF THE CHRISTENSENELLACEAE FOR USE AS A DRUG, IN PARTICULAR FOR TREATING OBESITY, CARDIOMETABOLIC DISEASES AND INFLAMMATORY BOWEL DISEASES

(30) Priorité: 10.03.2017 FR 1770237; 02.08.2017 FR 1770822
(43) Date de publication de la demande: 15.01.2020
(73) Titulaire: Ysopia Biosciences, 33000 Bordeaux (FR)
(72) Inventeur: VINCENT, Claude, 33000 Bordeaux (FR)
(74) Mandataire: Aquinov
(86) Numéro de dépôt international: PCT/EP2018/055937
(87) Numéro de publication internationale: WO 2018/162726

(56) Documents cités:
- WO-A1-2015/164555
- BEAUMONT MICHELLE ET AL: "Heritable components of the human fecal microbiome are associated with visceral fat", GENOME BIOLOGY, vol. 17, septembre 2016 (2016-09), XP002776389, cité dans la demande
- M. MOROTOMI ET AL: "Description of Christensenella minuta gen. nov., sp. nov., isolated from human faeces, which forms a distinct branch in the order Clostridiales, and proposal of Christensenellaceae fam. nov.", INTERNATIONAL JOURNAL OF SYSTEMATIC AND EVOLUTIONARY MICROBIOLOGY, vol. 62, no. 1, 25 février 2011 (2011-02-25), pages 144-149, XP055232975, GB ISSN: 1466-5026, DOI: 10.1099/ijs.0.026989-0
- PETER J. TURNBAUGH ET AL: "A core gut microbiome in obese and lean twins", NATURE, vol. 457, no. 7228, 22 janvier 2009 (2009-01-22), pages 480-484, XP055006664, ISSN: 0028-0836, DOI: 10.1038/nature07540 & DATABASE EMBL [Online] 16 décembre 2008 (2008-12-16), "Uncultured bacterium clone TS25_a01g01 16S ribosomal RNA gene, partial sequence.", extrait de EBI accession no. EM_STD:FJ366013 Database accession no. FJ366013

## Description

La présente invention se rapporte à des bactéries particulières pour leur utilisation comme médicament, en particulier pour prévenir ou lutter contre le surpoids, l'obésité, les maladies cardiométaboliques et les maladies inflammatoires de l'intestin. L'invention vise également des compositions incluant de telles bactéries.

Le surpoids et l'obésité touchent aujourd'hui toutes les populations, aussi bien dans les pays développés et que dans les pays en voie de développement. Ils sont à l'origine de maladies chroniques, telles que majoritairement les maladies cardiométaboliques en particulier le diabète et les maladies cardiovasculaires mais également certains cancers, les maladies neurodégénératives et les maladies inflammatoires intestinales, qui touchent une large portion de la population mondiale et deviennent la première cause de mortalité.

La médecine moderne se trouve démunie face à ces pathologies chroniques dont la prise en charge est plus difficile, chronophage et moins dichotomique que les maladies aigues. En outre, la pérennité de ces maladies dans le temps, avec le vieillissement de la population, a un impact considérable sur les dépenses de santé publiques qui deviennent insupportables. C'est pourquoi un premier objectif de l'invention est de proposer une solution simple, efficace et économique pour faire face à cette problématique de santé majeure.

La pandémie de ces maladies chroniques semble liée à une conjonction de plusieurs facteurs : un mauvais régime alimentaire type « western diet », la sédentarité et des facteurs touchant la génétique et l'épigénétique.

Les recherches pour lutter contre ces pathologies ont porté dans un premier temps sur les déséquilibres de la prise alimentaire, tant quantitatifs que qualitatifs (en particulier une forte consommation de lipides saturés, de fructose et d'hydrates de carbones) associés ou non à la sédentarité, qui conduisent à la prise de poids. Toutefois, il a été constaté que chacun n'est pas égal devant la même prise alimentaire et des recherches ont été menées pour évaluer l'impact de la génétique et épigénétique sur la prise de poids. Ces recherches ont montré que seule une infime part des problèmes de surpoids et d'obésité étaient attribuée d'une manière directe à la génétique. Par contre, les résultats au niveau de l'épigénétique ont permis de révéler un intérêt pour le microbiome qui semblait jouer un rôle important dans de nombreuses maladies chroniques et en particulier de l'obésité et des maladies cardiométaboliques. Il a notamment été constaté qu'une dysbiose intestinale quantitative mais surtout fonctionnelle pouvait entraîner une augmentation de l'absorption et du stockage de l'énergie sous forme de gras, à quantité égale de prise alimentaire. Cette dysbiose fonctionnelle est due à la disparition ou à la marginalisation de certaines bactéries qui ont une influence positive sur la santé de l'hôte . La plupart de ces bactéries sont héritables; ceci expliquant l'existence de lignées de maigres ou de gros, ou l'existence d'obèses qui n'ont jamais de dérèglements métaboliques, en particulier au niveau de la glycémie et des lipides, car ils n'ont pas de dysbiose intestinale fonctionnelle, les gènes de l'hôte ne subissant pas de mutation.

En effet, si l'existence d'une interaction microbiome/cerveau a été le premier pas pour comprendre l'action du microbiome en particulier dans le cadre du surpoids à travers des récepteurs de la satiété notamment, la découverte la plus importante a été celle de l'héritabilité des bactéries. Cette notion d'héritabilité a été démontrée par l'étude de cohortes de jumeaux monozygotiques (Goodrich « Genetic determinant of the gut microbiome in UK twins », CellPress 2016), et a permis de donner une explication à la transmission de l'obésité ou de certaines maladies chroniques. Ainsi dans le cadre de l'obésité et des maladies cardiovasculaires il a été découvert que des variations de gènes étaient liées directement au gras viscéral expliquant les épidémies d'obésité et de syndrome métabolique en particulier du diabète et des maladies cardiovasculaires, et ce aussi bien dans les pays développés que dans les pays émergents comme la Chine et les pays d'Amérique du Sud. Cette nouvelle notion d'épigénétique a bouleversé la compréhension de nombreuses maladies chroniques et permet d'envisager de nouveaux traitements grâce aux bactéries qui peuvent pallier aux changements génétiques. Ceci est d'autant plus important qu'il est prouvé maintenant que l'épigénétique de la mère et du père peut être transmise aux enfants particulièrement dans l'obésité.

Un autre objectif de l'invention, est par conséquent de proposer une solution pour lutter en particulier contre le surpoids, l'obésité, les maladies cardiométaboliques et les maladies inflammatoires de l'intestin en agissant sur le microbiome intestinal et en particulier sur des bactéries héritables.

Le microbiome est un ensemble de microorganismes (bactéries, archées, virus et eucaryotes regroupés en domaines) qui sont spécifiques à chaque individu. Ces microorganismes se trouvent sur la peau, dans la bouche et le plus grand nombre dans le système digestif qui en compte environ 3,3 millions. Ce microbiome contient ainsi plus de cent cinquante fois de gènes que le génome humain.

Les différents niveaux pour classer les bactéries sont : domaine, phylum, classe, ordre, famille, genre, espèce et souche. Les bactéries sont également identifiables et caractérisables par UTOs (Unités Taxonomiques Opérationnelles), une UTO correspondant à un regroupement de souches de bactéries d'une même espèce dont les séquences d'ARNr 16S présentent une similitude de plus de 97%.

Pour connaître et analyser les bactéries constituants le microbiome intestinal, plusieurs méthodes existent actuellement :
- La culture : cette technique est limitée car 80% du microbiome n'est pas cultivé (actuellement seules mille espèces sont cultivées en raison des difficultés à pratiquer cette culture),
- Le marquage biologique en utilisant comme marqueur l'ARNr 16S
- Le séquençage d'UTO soit par la méthode SANGER soit par pyroséquençage de l'ARNr 16S,
- L'empreinte par électrophorèse, polymorphisme et ribosomal
- Les puces à ADN
- Les méthodes FISH et qPCR avec amplification d'un groupe
- L'analyse métagénomique shootgun
- La méta analyse avec la métagénomique (composition et fonction de tous les gènes), métaprotéomique (analyse des protéines), métabolomique (profil métabolique) et métatranscriptomique (ARN).

Toutefois, actuellement, parmi les millions de bactéries constituant le microbiome, très peu (un millier environ) sont définies, caractérisées et cultivées.

Chaque individu a son propre microbiome qui provient de son histoire et de ses racines, mais il existe des espèces communes à de nombreux individus. En particulier, on sait que plus de 85% des espèces sont communes entre l'Europe, les Etats-Unis et le Japon.

Par ailleurs, le microbiome intestinal n'est pas homogène. Il varie en quantité et qualité lorsqu'il s'agit de l'estomac, du duodénum, jéjunum, iléum et enfin du colon où se trouve le plus grand nombre de bactéries anaérobiques. On sait également que certaines bactéries se trouvent dans le mucus qui tapisse la paroi intestinale.

De plus le système immunitaire, le système génétique et les neurotransmetteurs (en particulier ceux faisant la liaison entre intestin et cerveau) influencent le microbiome ce qui rend son étude encore plus complexe.

Il s'agit donc d'un domaine particulièrement délicat par sa complexité et le nombre de facteurs inter-réagissant et il est impossible aujourd'hui d'établir un microbiome standard de bonne santé.

Dans le surpoids et l'obésité, on sait que la diversité du microbiome diminue ainsi que sa richesse (61% des obèses ont un nombre bas de genres). Une corrélation entre certains phyla (notamment firmicutes, bacteriodetes, protéobacteria et actinobacteria) et le surpoids et/ou l'obésité a été recherchée. Une relation entre le rapport firmicutes sur bacteriodetes a été décrite comme augmentant avec l'IMC (indice de masse corporelle), mais avec la réplication des études, certains scientifiques ont infirmé ce jugement en démontrant le contraire. Ainsi, rester au niveau de grands ensembles comme les phyla n'apporte pas de solution pour lutter contre le surpoids et/ou l'obésité et les maladies métaboliques chroniques. Les recherches se sont orientées vers des niveaux plus bas dans la classification bactérienne, au niveau des familles, des genres, des espèces voire des souches.

De nombreuses études ont été réalisées au niveau des familles de bactéries, mais là encore aucun résultat satisfaisant n'a été obtenu. En effet, une famille peut contenir des dizaines d'espèces et encore plus de souches, sans possibilité de savoir quelle(s) bactérie(s) possède(nt) l'action. De plus, certaines familles peuvent abriter des espèces dangereuses à côté d'espèces bénéfiques.

Récemment, des études ont été réalisées sur des espèces susceptibles d'influencer la perte de poids par leur augmentation dans le microbiome, à savoir *Akkermansia muciniphila, Methanobrevibacter smithii, Faecalibacterium prausnitzii, Bifidobacterium longum, Roseburia intestinalis, Eubacterium rectale* et *Christensenella minuta.* Toutefois, les études ont été réalisées uniquement sur la souris ce qui ne laisse pas présager une quelconque activité sur l'homme. Les études sur la souris ne sont pas fiables car ces animaux possèdent un microbiome différent de l'homme et les souris à microbiome humanisé perdent leurs réactions naturelles sur les marqueurs métaboliques.

Concernant les maladies cardiométaboliques comme le diabète, il a été montré qu'il peut exister in vitro ou sur l'animal, une baisse importante des Clostridiales et une augmentation des Bacteriodetes, ainsi qu'une baisse de *Roseburia intestinalis* et *Faecalibacterium pransnitzii,* qui réguleraient le glucose et la glycémie à travers la perméabilité de la barrière intestinale, mais là encore ces constats ne permettent pas d'amener une solution satisfaisante pour lutter contre le surpoids, l'obésité et les maladies cardiométaboliques en découlant car le nombre de bactéries différentes est trop important.

Trois types de thérapies bactériennes agissant sur le microbiome ont été proposées à ce jour.

La première a été d'utiliser les prébiotiques pour augmenter l'ensemble des bactéries afin de compenser la diminution induite par l'obésité. Toutefois cette solution est au mieux un leurre et au pire un danger en faisant augmenter les bactéries non souhaitées ce qui est la tendance naturelle du microbiome en dysbiose. Une croissance sélective a également été recherchée in vitro en croisant nourriture sélectionnée sur souche sélectionnée mais cela n'a pas fonctionné chez l'homme.

La deuxième a consisté à utiliser les probiotiques comme « nourriture » de développement de bactéries sélectionnées. Cette solution n'est pas non plus satisfaisante car les probiotiques développés ont été limités aux bactéries alimentaires qui sont utilisées dans la première partie de l'intestin alors que les bactéries intéressantes sur le plan métabolique sont dans le colon et nichées dans le mucus. De plus, il est difficile de démontrer une action sur l'homme à long terme car l'activité est hautement aléatoire.

Enfin, la troisième solution proposée est l'implantation fécale de bactéries de personnes normales ou maigres à des personnes obèses selon une technique qui fonctionne sur les souris. Cette technique présente l'inconvénient d'essayer d'implanter des souches étrangères qui peuvent être rejetées et il existe une transmission possible de signaux cancérigènes selon les craintes exprimées par les cancérologues.

Il n'existe donc pas aujourd'hui de solution thérapeutique agissant sur le microbiome qui soit satisfaisante pour l'homme en surpoids, obèse et/ou souffrant de maladies cardiométaboliques et/ou de maladies inflammatoires de l'intestin.

L'invention a donc pour objectif de répondre à ce besoin, en proposant d'utiliser de nouvelles bactéries spécifiques agissant de façon particulièrement efficace pour lutter contre le surpoids, l'obésité, les maladies cardiométaboliques et les maladies inflammatoires de l'intestin.

A cet effet, l'invention porte sur l'utilisation de bactéries particulières sélectionnées et récoltées à partir du microbiome de personnes en surpoids ou obèses et présentant un syndrome métabolique. En particulier l'invention vise des bactéries commensales, héritables, gram négatif, strictement anaérobiques et non sporulées de la famille des *Christensenellacées* et appartenant à une UTO caractérisée par une séquence d'ARNr 16S SEQ ID N°1.

Des bactéries de la famille des *Christensenellacées,* notamment du genre *Christensenella,* ont déjà été étudiées, notamment dans la demande US2017042948. C'est le cas en particulier de *Christensenella minuta, Christensenella massiliensis* et *Christensenella timonensis.* On sait également que les personnes en surpoids ou obèse présentant une dysbiose en bactéries héritables de la famille des *Christensenellacées* connaissent une variation rs74331972 de leur gène FIHT et une variation rs 1433723 de leur gène TDRG1 (Beaumont « Christensenella, heridity and visceral fat heritable components », Genome biology 2016).

Toutefois, toutes les bactéries de la famille des Christensenellacées ne jouent pas un rôle dans le surpoids, l'obésité et/ou les maladies cardiométaboliques et/ou inflammatoires, et aucune des bactéries précitées n'a démontré de lien direct avec ces pathologies chez l'homme.

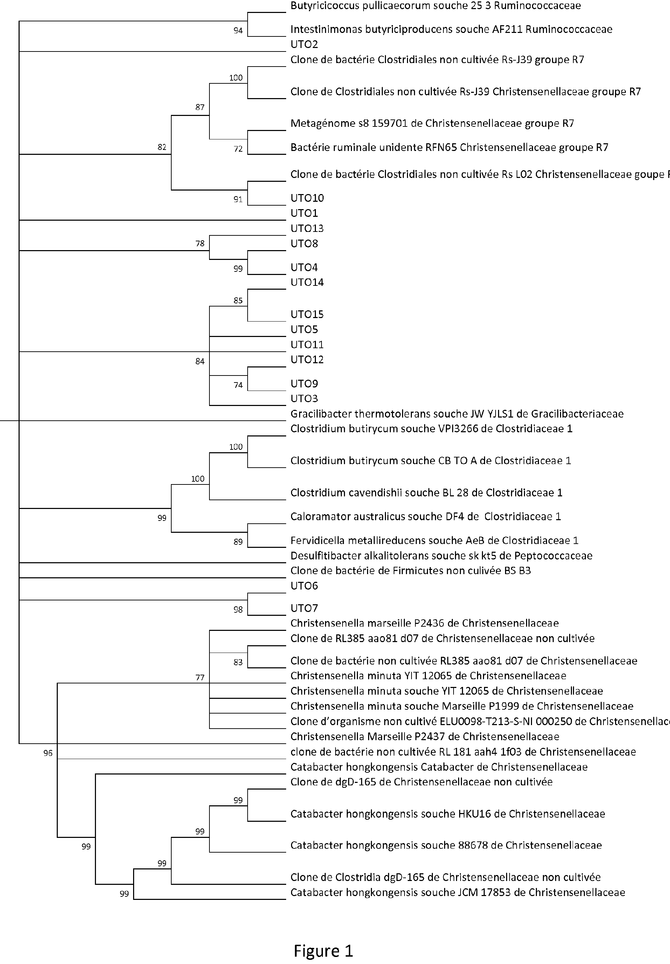

Les bactéries selon l'invention sont éloignées (voir arbre phylogénétique Figure 1) de celles déjà décrites pour leur utilisation dans la lutte contre le surpoids, l'obésité, les maladies cardiométaboliques et/ou les maladies inflammatoires de l'intestin.

Avantageusement les bactéries selon l'invention présentent une efficacité significativement démontrée et très importante pour lutter contre le surpoids, l'obésité, les maladies cardiométaboliques et les maladies inflammatoires de l'intestin chez l'homme, en particulier en diminuant le gras viscéral d'une part et la perméabilité intestinale d'autre part. Elles ont été sélectionnées de façon spécifique à partir d'une population de personnes en surpoids ou obèses avec syndrome métabolique et sont par conséquent parfaitement adaptées pour une utilisation comme médicament efficace chez l'homme avec une grande adaptabilité pour une implantation dans un milieu inflammatoire.

L'invention a également pour objet des compositions comprenant ces bactéries. telles que définies dans les revendications.

D'autres caractéristiques et avantages de l'invention ressortiront de la description en détail de l'invention qui va suivre réalisée en regard notamment de la Figure 1 qui représente l'arbre phylogénétique des bactéries selon l'invention.

### DEFINITIONS

Par « ARNr » on entend l'acide ribonucléique ribosomique.

Par «ARNr 16S» on entend l'ARNr constituant la petite sous-unité des ribosomes des procaryotes. Chez les bactéries, leur séquence permet d'identifier et de caractériser des unités taxonomiques opérationnelles.

Par « obésité » on entend une concentration de masse graisseuse trop élevée dans l'organisme avec une augmentation du volume du tissu adipeux, tissu contenant ces graisses. Les êtres humains adultes obèses présentent un IMC (indice de masse corporelle) supérieur à 30.

Par « maladie cardiométabolique » on entend une maladie métabolique et/ou cardiovasculaire. Ces maladies cardiométaboliques peuvent être induites par le surpoids et/ou l'obésité. Il peut s'agir en particulier du pré-diabète, du diabète, des maladies vasculaires et cardiaques, de l'athérosclérose, de l'hyperlipidémie, de l'hyperglycémie, du NASH (stéatose hépatique non alcoolique), du NAFLD (maladie du foie gras non alcoolique), des infarctus, des ictus, de l'hypertension.

Par « utilisation comme médicament » on entend une utilisation pour tout effet curatif ou préventif à l'égard des maladies humaines ou animales, ainsi que toute utilisation chez l'homme, en vue d'établir un diagnostic médical ou de restaurer, corriger ou modifier leurs fonctions organiques. L'utilisation comme médicament dans le cadre de la présente invention inclut notamment une utilisation comme modulateur spécifique du microbiome pour reconstituer un écosystème bénéfique pour la santé de l'hôte.

Par « risque cardiométabolique » ou « syndrome métabolique » on entend un syndrome qui regroupe au moins trois facteurs de risque choisis notamment parmi : tour de taille, glycémie, HDL cholestérol, triglycérides et tension artérielle, et qui conduit à des maladies cardiométaboliques.

Par « surpoids » on entend une concentration de masse graisseuse trop élevée dans l'organisme avec une augmentation du volume du tissu adipeux, tissu contenant ces graisses. Les êtres humains adultes en surpoids présentent un IMC (indice de masse corporelle) supérieur à 25.

Par « transplant fécal » on entend des selles destinées à être transplantés dans un individu et provenant soit de l'individu lui-même prélevés avant traitement et implantés après traitement, soit d'un autre individu réputé sain.

Par « UTO » ou « Unité Taxonomique Opérationnelle » on entend un regroupement de bactéries d'une même espèce dont les séquences d'ARNr 16S présentent une similitude de plus de 97%. Ainsi une UTO caractérisée par une séquence d'ARNr 16S « X », comprend les bactéries avec une séquence d'ARNr 16S présentant une similitude de plus de 97,5% avec la séquence « X ».

### DESCRIPTION DETAILLEE DE L'INVENTION

L'invention a pour objet une bactérie commensale, héritable gram négatif, strictement anaérobique et non sporulée de la famille des *Christensenellacées* et appartenant à une UTO caractérisée par une séquence d'ARNr 16S SEQ ID N°1 (dénommée UTO 1 pour la présente description), pour une utilisation comme médicament.

La bactérie OTU1 peut être utilisée avec une bactérie commensale, héritable gram négatif, strictement anaérobique et non sporulée de la famille des *Christensenellacées* et appartenant à une UTO caractérisée par une séquence d'ARNr 16S SEQ ID N°2 (dénommée UTO 2 pour la présente description).

Les bactéries UTO 1 et UTO 2 sont des bactéries commensales et héritables présentes dans le mucus intestinal humain.

Elles présentent préférentiellement un diamètre de 0,5µm.

Elles peuvent être notamment cultivées dans un pH compris entre 7 et 8 dans des conditions anaérobiques strictes, à une température de 37°C pendant 4 jours sur une levure avec une supplémentation de glucose nourricier.

Elles sont petites productrices d'acides gras à courtes chaînes, en particulier d'acide acétique, d'isobutyrate et d'isovalérate.

Les séquences d'ARNr 16S qui permettent d'identifier spécifiquement et de façon certaine ces bactéries, sont éloignées des séquences d'ARNr 16S de bactéries connues de la même famille et du même genre telles que *Christensenella minuta, Christensenella massiliensis, Christensenella timonensis.* Elles sont en dessous de 90% de *Christensenella minuta* dans l'arbre phylogénétique (voir Figure 1) et sont bien individualisées.

Les bactéries selon l'invention peuvent être prélevées et sélectionnées à partir du microbiome intestinal d'une population de personnes en surpoids ou obèses avec syndrome métabolique. Elles sont corrélées à l'amélioration de nombreux facteurs caractéristiques du surpoids, de l'obésité, des maladies cardiométaboliques ou des maladies inflammatoires de l'intestin, et en particulier à une diminution du gras viscéral. Les bactéries selon l'invention peuvent être récoltées notamment par prélèvement de matière fécale en particulier de matière fécale d'une personne en surpoids ou obèse et en syndrome métabolique dont les gènes FIHT et TDRG1 n'ont pas subi de variation génétique, et plus précisément dont le gène FIHT n'a pas subi une variation rs74331972 et dont le gène TDRG1 n'a pas subi de variation rs 1433723.

Les bactéries utiles selon l'invention peuvent être administrées à l'homme par tout mode d'administration adapté, en particulier par voie orale ou par voie rectale.

Elles sont particulièrement utiles dans le traitement du surpoids, de l'obésité et/ou dans le traitement des maladies cardiométaboliques et/ou des maladies inflammatoires de l'intestin, en particulier des maladies cardiométaboliques et/ou inflammatoires de l'intestin induites par le surpoids et l'obésité.

Avantageusement, les bactéries selon l'invention sont notamment capables de :
- diminuer le poids, et/ou
- diminuer le gras viscéral et le gras sous-cutané, et/ou
- préserver la masse maigre, et/ou
- diminuer les graisses hépatiques et bloquer la fibrose du foie, et/ou
- diminuer le tour de taille, et/ou
- normaliser la tension artérielle, et/ou
- diminuer l'inflammation de la barrière intestinale, des organes et des tissus musculaires ou adipeux, en particulier du colon, et/ou
- diminuer la perméabilité de la barrière intestinale par l'action sur la reconstitution du mucus, la fermeture des jonctions serrées, la réactivation des récepteurs hormonaux comme notamment TLR4, GPL2, CCK et/ou
- réguler le cholestérol HDL, LDL et cholestérol total et/ou
- réguler les triglycérides, et/ou
- diminuer la glycémie et/ou la glycémie post-prandiale, et/ou l'insulino-résistance et/ou l'insulino-sensibilité.
- diminuer l'inflammation de l'intestin, en particulier dans les maladies intestinales inflammatoires et notamment la maladie de Crohn (la maladie de Crohn étant liée au syndrome métabolique et donc au même microbiome).

Ces différents facteurs sont des caractéristiques ou des conséquences des maladies cardiométaboliques ou des maladies inflammatoires de l'intestin, en particulier chez les personnes en surpoids ou obèses, si bien que l'action des bactéries selon l'invention sur ces facteurs permet de lutter contre le surpoids, l'obésité, les maladies cardiométaboliques et les maladies inflammatoires de l'intestin.

Les bactéries selon l'invention sont par conséquent particulièrement utiles dans le traitement du surpoids, de l'obésité, du syndrome métabolique, du pré-diabète, du diabète, des maladies vasculaires et cardiaques, de l'athérosclérose, de l'hyperlipidémie, de l'hyperglycémie, du NASH (stéatose hépatique non alcoolique) et/ou du NAFLD (maladie du foie gras non alcoolique) et/ou des MICI (maladies inflammatoires chroniques intestinales ou en anglais IBD « Inflammatory Bowel Disease ») en particulier la maladie de Crohn. Avantageusement l'invention permet d'agir sur la reconstitution du mucus, de l'épithélium et l'augmentation de l'imperméabilité de la barrière intestinale pour la modulation des jonctions serrées afin de diminuer l'endotoxémie et la translocation sanguine des bactéries et des endotoxines lipopolysaccharides (LPS). En effet, les personnes en surpoids et les personnes obèses et celles présentant des maladies cardiométaboliques et/ou des maladies inflammatoires de l'intestin, ont un mucus dégradé et mince qui se renouvelle mal. De plus, l'épithélium enflammé est déficient aves des jonctions serrées très ouvertes qui laissent passer les bactéries et les LPS. Ce phénomène est dû à l'inflammation de l'intestin et il provoque l'inflammation chronique de bas grade dans l'organisme.

Les bactéries utiles selon l'invention sont particulièrement adaptées aux personnes présentant un IMC supérieur à 25 et en particulier supérieur à 30 et/ou à des personnes présentant une hypertriglycémie.

Avantageusement, l'utilisation des bactéries UTO 1 et/ou UTO 2 permet d'augmenter la population bactérienne efficiente de la famille *Christensenellaceae* sans augmenter les bactéries potentiellement dangereuse comme *Catabacter hongkongensis* de la même famille et proche de *Christensenella minuta*.

Les bactéries selon l'invention peuvent être utilisées en même temps qu'un rééquilibrage alimentaire et/ou une restriction calorique. Le rééquilibrage alimentaire peut consister en particulier à éliminer de l'alimentation des nutriments délétères pour le microbiome : le fructose et des acides gras trans. La restriction calorique est préférentiellement légère, d'environ 600 kcal/jour. Elle permet notamment de moduler l'homéostasie du glucose, le turnover des protéines et la lipogenèse.

Les bactéries selon l'invention peuvent être utilisées à une dose unitaire quotidienne de 10⁴ à 10¹² (en nombre de bactéries), préférentiellement entre10⁴ à 10¹².

Préférentiellement les bactéries selon l'invention sont administrées à une fréquence quotidienne à hebdomadaire. Une telle fréquence permet de conserver une colonie de bactéries selon l'invention suffisante dans le microbiome et éviter que la mortalité des bactéries soit supérieure aux bactéries en croissance.

La détermination de la fréquence se fait selon plusieurs critères en particulier l'importance du symptôme à traiter, l'importance du gras viscéral à perdre, l'importance de l'hyperglycémie ou hypertriglycérémie ou la fréquence des diarrhées.

La cure durera jusqu'à l'obtention du résultat escompté ; un traitement d'entretien peut être préconisé pour des cas où l'implantation des bactéries a été longue.

Une analyse des selles ou un prélèvement de mucus pour vérifier la teneur en UTO 1 et/ou en UTO 2 n'est pas obligatoire pour tous les patients en début de traitement, car il s'agit d'une bactérie héritable liée directement au gras viscéral. Toutefois, elle peut être nécessaire pour les patients avec un IMC très haut ou un inflammation élevée (CRP us). De même, si après plusieurs mois de traitement le résultat en fonction des effets recherchés (diminution du tour de taille, perte de poids, diminution de l'hyperglycémie etc.) n'est pas atteint, il est préférable de réaliser une analyse des selles pour vérifier que les bactéries ont bien niché ou si des facteurs génétiques ou épigénétiques fortement perturbés nécessitent l'utilisation d'une fréquence d'administration plus importante que celle prévue initialement. Dans tous les cas une analyse des selles ou un prélèvement direct du mucus est préférable après un an de la fin du traitement.

L'avantage de bactéries de la famille *Christensenellacées* est d'avoir une bonne corrélation entre la présence de ces bactéries dans les selles et dans le mucus ce qui n'oblige pas à faire des prélèvements par endoscopie dans le colon.

De façon avantageuse, les bactéries selon l'invention peuvent être utilisées dans le cadre d'une médecine personnalisée. En effet, il est possible d'adapter le traitement à la génétique du patient pour savoir dans quelle mesure il répondra au traitement et quelle est la dose à utiliser. Dans le cadre des bactéries héritables de la famille des *Christensenellacées* les gènes qui connaissent une variation sont connus il s'agit de FHIT avec la variation rs 74331972 et TDRG1 variante rs 1433723. Il est ainsi possible d'utiliser une analyse génétique de l'hôte en parallèle de l'analyse génétique du microbiome pour personnaliser totalement le traitement du patient dans des cas graves avec une éventuelle résistance au traitement afin d'en ajuster la dose à administrer.

Les bactéries selon l'invention peuvent être utilisées seules directement, mais préférentiellement, elles sont utilisées au sein d'une composition.

L'invention a donc également pour objet une composition comprenant au moins une UTO 1 ou au moins une UTO 2, c'est à dire au moins une bactérie commensale héritable gram négatif, strictement anaérobique et non sporulée de la famille des *Christensenellacées,* et appartenant à une UTO caractérisée par une séquence d'ARNr 16S SEQ ID N°1 ou au moins une bactérie commensale héritable gram négatif, strictement anaérobique et non sporulée de la famille des *Christensenellacées,* et appartenant à une UTO caractérisée par une séquence d'ARNr 16S SEQ ID N°2.

La composition comprend également des excipients, connus de l'homme du métier et classiquement utilisés dans l'industrie pharmaceutique, à adapter en fonction de la forme galénique de la composition.

Selon un mode de réalisation particulièrement adapté, la composition selon l'invention comprend l'association :
- d'au moins une bactérie commensale, héritable, gram négatif, strictement anaérobique et non sporulée de la famille des *Christensenellacées* et appartenant à une UTO caractérisée par une séquence d'ARNr 16S SEQ ID N°1 (UTO 1), et
- d'au moins une bactérie commensale héritable, gram négatif, strictement anaérobique et non sporulée de la famille des *Christensenellacées* et appartenant à une UTO caractérisée par une séquence d'ARNr 16S SEQ ID N°2 (UTO 2).

Avantageusement, l'association des bactéries selon l'invention appartenant à UTO 1 et des bactéries selon l'invention appartenant à UTO 2 permet d'obtenir un effet plus important sur les applications thérapeutiques recherchées.

Les bactéries UTO 1 et/ou UTO 2, peuvent également être associées à d'autres bactéries dans la composition.

La composition peut notamment également comprendre au moins une bactérie commensale, héritable, de la famille des *Christensenellacées* et appartenant à une UTO (dénommée UTO 3 pour la présente description) caractérisée par une séquence d'ARNr 16S SEQ ID N°3 et/ou au moins une bactérie appartenant à une UTO (dénommée UTO 4 pour la présente description) caractérisée par une séquence d'ARNr 16S SEQ ID N°4 et/ou au moins une bactérie appartenant à une UTO (dénommée UTO 5 pour la présente description) caractérisée par une séquence d'ARNr 16S SEQ ID N°5 et/ou au moins une bactérie appartenant à une UTO (dénommée UTO 6 pour la présente description) caractérisée par une séquence d'ARNr 16S SEQ ID N°6 et/ou au moins une bactérie appartenant à une UTO (dénommée UTO 7 pour la présente description) caractérisée par une séquence d'ARNr 16S SEQ ID N°7 et/ou au moins une bactérie appartenant à une UTO (dénommée UTO 8 pour la présente description) caractérisée par une séquence d'ARNr 16S SEQ ID N°8 et/ou au moins une bactérie appartenant à une UTO (dénommée UTO 9 pour la présente description) caractérisée par une séquence d'ARNr 16S SEQ ID N°9, et/ou au moins une bactérie appartenant à une UTO (dénommée UTO 10 pour la présente description) caractérisée par une séquence d'ARNr 16S SEQ ID N°10, et/ou au moins une bactérie appartenant à une UTO (dénommée UTO 11 pour la présente description) caractérisée par une séquence d'ARNr 16S SEQ ID N°11, et/ou au moins une bactérie appartenant à une UTO (dénommée UTO 12 pour la présente description) caractérisée par une séquence d'ARNr 16S SEQ ID N°12, et/ou au moins une bactérie appartenant à une UTO (dénommée UTO 13 pour la présente description) caractérisée par une séquence d'ARNr 16S SEQ ID N°13, et/ou au moins une bactérie appartenant à une UTO (dénommée UTO 14 pour la présente description) caractérisée par une séquence d'ARNr 16S SEQ ID N°14, et/ou au moins une bactérie appartenant à une UTO (dénommée UTO 15 pour la présente description) caractérisée par une séquence d'ARNr 16S SEQ ID N°15.

Ces bactéries sont capables d'agir sur les mêmes facteurs que les bactéries utiles selon l'invention, mais peuvent difficilement être utilisées seules car elles sont moins fréquentes dans le microbiome et ainsi leur implantation est plus aléatoire. En revanche leur utilisation en combinaison avec les bactéries selon l'invention permet d'améliorer l'efficacité de ces dernières et renforce l'ecosystème des *Christensenellacées.* Elles peuvent être sélectionnées de la même façon que l'UTO 1 et/ou l'UTO 2.

La composition selon l'invention peut comprendre aussi une ou plusieurs bactéries méthanogènes de la famille des *Methanobacteriaceae,* du genre *Methanobacterium* et/ou *Methanobrevibacter* et/ou *Methanosphaera* et/ou *Methanothermobacter,* en particulier *Methanothermobacter smithii,* Les méthanogènes facilitent l'implantation des *Christensenellacées* en particulier chez les obèses ou les personnes souffrant de diabètes mais par contre doivent être utilisés avec précaution dans les maladies inflammatoires de l'intestin.

Elle peut également comprendre une ou plusieurs bactéries des espèces, genres ou ordres suivants : *Marvinbryantia formatexigens* et/ou *Bacteroides thetaiotaomicron* et/ou *Akkermansia muciniphila* et/ou *Faecalibacterium prausnitzii* et/ou *Clostridium thermocellum* et/ou *Dehalobacteriaceae et*/*ou Oscillospira et*/*ou Mogibacteriaceae et*/*ou Ruminococcaceae et*/*ou Ruminococcus et*/*ou Lachinospiraceae et*/*ou Lachinospira et*/*ou Bacteroidaceae et*/*ou Rikenellaceae et*/*ou Clostridium et*/*ou Clostridiales.* Toutes ces bactéries peuvent être des bactéries des souches connues actuellement cultivées ou peuvent être de nouvelles souches, en particulier des souches récoltées à partir des matières fécales d'une personne en surpoids ou obèse et en syndrome métabolique pour obtenir des souches résistantes à l'inflammation caractéristique des personnes en surpoids ou obèses. Ces bactéries sont connues pour avoir une influence sur la régulation du poids chez la souris même si la plupart ne sont pas héritables donc avec un mode d'action inconnu. L'hypothèse scientifique la plus probable est qu'elles renforcent l'écosystème de la famille *Christensenellacées.*

Les bactéries utiles selon l'invention peuvent aussi être utilisées en combinaison avec au moins une autre bactérie choisie parmi *Christensenella massiliensis, Christensenella timonensis, Christensenella minuta.* Là encore ces bactéries peuvent être récoltées à partir des matières fécales d'une personne en surpoids ou obèse et en syndrome métabolique pour obtenir des souches résistantes à l'inflammation caractéristique des personnes en surpoids ou obèses. Ces bactéries sont de la famille *Christensenellacées* avec une action moins probante dans l'expérience clinique sur les patients obèses avec un risque cardiométabolique mais elles permettent de créer à la fois une synergie mais surtout un renforcement de l'écosystème des bactéries de l'invention.

Les compositions selon l'invention peuvent également comprendre d'autres constituants, en particulier au moins un acide aminé et/ou au moins un peptide. Il peut s'agir en particulier de la thréonine, la leucine, la sérine, la proline, l'alanine, la glycine, la glutamine, l'acide glutamique, le tryptophane et/ou d'au moins un peptide contenant au moins un de ces acides aminés. Selon une variante ces acides aminés peuvent être administrés en parallèle des bactéries selon l'invention, mais dans une composition indépendante. La quantité de chacun des acides aminés ou biopeptides est préférentiellement comprise entre 500mg et 1g par jour.

La présence d'acide(s) aminé(s) ou de peptide(s) en plus des bactéries utiles selon l'invention permet notamment de renforcer le mucus intestinal et de restaurer la barrière intestinale en diminuant sa perméabilité. Ces 2 facteurs sont caractéristiques des personnes obèses et des malades cardiométaboliques, le mucus est plus mince empêchant la niche des bactéries commensales comme les bactéries selon l'invention; les jonctions serrées étant largement ouvertes permettant l'endotoxémie qui provoque l'inflammation chronique de l'organisme. Les compositions selon l'invention peuvent comprendre aussi au moins un probiotique et/ou au moins un prébiotique. Il peut s'agir par exemple d'oligosaccharides en particulier RS4 pour les prébiotiques ou des ferments lactiques pour les probiotiques. Les probiotiques permettent d'améliorer la dysbiose quantitative et la santé générale du microbiote. Les prébiotiques permettent d'augmenter la nourriture des bactéries selon l'invention qui ont été implantées. Préférentiellement les prébiotiques et/ou les probiotiques sont administrés au début du traitement avec les bactéries selon l'invention.

Les bactéries selon l'invention sont préférentiellement utilisées vivantes. Dans les compositions selon l'invention, les bactéries présentes (UTO 1, UTO 2 et éventuelles autres bactéries) sont de façon préférée pour au moins 50% des bactéries vivantes (pourcentage en nombre), encore plus préférentiellement au moins 90%.

Les bactéries utiles selon l'invention et les compositions les incluant peuvent se présenter sous toutes formes. Préférentiellement les bactéries et/ou la composition se présentent sous forme lyophilisée.

Préférentiellement, les compositions selon l'invention se présentent sous forme de poudre, de poudre microencapsulée, de gélule, de capsugel, de comprimé, ou de transplant fécal. Selon une variante particulièrement adaptée, les compositions selon l'invention se présentent sous une forme gastro-résistante, en particulier sous une forme gastro-résistante destinée à être absorbée au niveau intestinal de préférence au niveau du colon, en particulier au niveau de l'iléum terminal ou de l'entrée du colon.

Les compositions selon l'invention sont fabriquées par simple mélange des constituants et/ou par des méthodes classiques et connues de l'homme du métier adaptées aux formes galéniques souhaitées.

Les compositions selon l'invention peuvent être utilisées pour toutes les applications thérapeutiques, en particulier toutes les applications thérapeutiques des bactéries utiles selon l'invention telles que décrites dans la présente demande.

Préférentiellement, les compositions selon l'invention sont utilisées à une dose unitaire quotidienne pour chaque bactérie présente dans la composition (UTO1, UTO2 et autres bactéries éventuellement présentes) de 10⁴ à 10¹². Il est possible d'adapter la posologie des bactéries présentes dans la composition en fonction de plusieurs critères, en particulier en fonction de la quantité desdites bactéries retrouvée dans les selles prélevées chez les personnes à traiter.

Les bactéries et les compositions selon l'invention peuvent être données aussi bien aux adultes, qu'aux personnes âgées, aux enfants et aux adolescents. La posologie peut être la même pour tous les patients quel que soit l'âge.

De façon avantageuse, l'invention permet de diminuer les échecs thérapeutiques qui sont nombreux soit par l'abandon du traitement en cours et le plus souvent au début, soit par la reprise de poids après quelques mois et avec une reprise plus importante qu'auparavant de masse grasse viscérale, soit par la continuation de maladies chroniques cardiométaboliques et/ou inflammatoires et de leurs manifestations aigues.

L'invention est à présent illustrée par des exemples de compositions selon l'invention et par une étude clinique démontrant l'efficacité et l'intérêt des bactéries selon l'invention.

### EXEMPLES DE COMPOSITIONS SELON L'INVENTION

### Exemple 1

Cet exemple de composition selon l'invention est une composition sous forme lyophilisée comprenant l'UTO 1 et au moins un excipient adapté.

### Exemple 2

Cet exemple de composition selon l'invention est une composition sous forme lyophilisée comprenant l'UTO 1 et l'UTO 2 et au moins un excipient adapté.

### Exemple 3

Cet exemple de composition selon l'invention est une composition sous forme lyophilisée comprenant l'UTO 1, l'UTO 2, l'UTO3, l'UTO 4, l'UTO 5, l'UTO 6, l'UTO 7, l'UTO 8, l'UTO 9, l'UTO 10, l'UTO 11, l'UTO 12, l'UTO 13, l'OTU14 et l'OTU15 et au moins un excipient adapté.

### Exemple 4

Cet exemple de composition selon l'invention est une composition sous forme lyophilisée comprenant l'UTO 1, l'UTO 2 et *Akkermansia muciniphali* et au moins un excipient adapté.

### Exemple 5

Cet exemple de composition selon l'invention est une composition sous forme lyophilisée comprenant l'UTO 1, l'UTO 2 et *Methanobrevibacter smithii* et au moins un excipient adapté.

### Exemple 6

Cet exemple de composition selon l'invention est une composition sous forme lyophilisée comprenant l'UTO 1, l'UTO 2, *Christensenella massiliensis, Christensenella timonensis,* et *Christensenella minuta* et au moins un excipient adapté.

### Exemple 7

Cet exemple de composition selon l'invention est une composition sous forme lyophilisée comprenant l'UTO 1, l'UTO 2, *Faecalibacterium prausnitzii, Oscillospira et Lachinospira* et au moins un excipient adapté.

### EVALUATION DE L'EFFET DE L'INVENTION

Une étude clinique interventionnelle a été réalisée en France sur une population de 107 personnes obèses ou en surpoids et en syndrome métabolique avec une obésité abdominale (tour de taille supérieur à 86cm pour les femmes et 94cm pour les hommes) et deux autres facteurs de risques du syndrome métabolique.

Un produit connu pour lutter contre le surpoids, l'obésité et le syndrome métabolique a été administré accompagné d'une restriction calorique de 600 kcal pour une durée de 12 semaines.

Les selles des patients ont été prélevées au début et à la fin de l'étude après 12 semaines, pour déceler les impacts sur les corrélations entre les bactéries présentes dans les selles et l'évolution des signes cliniques. Le critère principal retenu de l'évolution du syndrome métabolique était la masse grasse viscérale mesurée par CTScan et le poids en critère secondaire.

La détermination et la caractérisation des bactéries a été réalisée par analyse de l'ARNr 16S, confirmée par qPCR et une métagénomique shotgun.

Il s'est avéré que seules 286 UTO étaient significativement liées à la perte de gras viscéral (p=0.002) et de poids sur l'homme. Ces bactéries appartiennent à l'ordre des Clostridiales et à la famille des *Christensenellacées.* Parmi ces 286 UTO, 15 d'entre elles avaient une représentativité suffisante chez les patients étudiés et une corrélation positive avec la perte de gras viscéral. Il a également été noté que l'action de ces bactéries était plus importante sur les patients ayant au départ une hypertriglycérémie. Ces UTOs sont les UTO 1 à 15 telles que décrites dans la présente demande.

La quantité de départ de ces UTOs et leur évolution après traitement est présentée dans le Tableau 1 ci-après.

**Tableau 1**

| Bactéries | Nombre de patients modulés | % du nombre de patients | Quantité de départ de la bactérie (%) | Evolution (multiplié par) | P student | Δ% Poids | P student | Δ% Gras Viscéral | P Student |
|---|---|---|---|---|---|---|---|---|---|
| Population totale | 107 | | - | -- | -- | | | -9.4 | 0.0000 |
| OTU1 | 21 | 41 | 0.00043 | 5.88 | 0.0007 | -4,8 | 0,0000 | -12.8 | 0.0003 |
| OTU 2 | 19 | 37 | 0.00013 | 2.97 | 0.0002 | -4,8 | 0,0000 | -15.4 | 0.0003 |
| OTU 3 | 10 | 20 | 0.00198 | 2.16 | 0.0043 | -5,1 | 0,0008 | -19.5 | 0.0093 |
| OTU4 | 10 | 20 | 0.00019 | 3.39 | 0.0354 | -5,1 | 0,0019 | -16.4 | 0.0136 |
| OTU 5 | 10 | 20 | 0.00001 | 54.78 | 0.2932 | -4,2 | 0,0125 | -17.7 | 0.0035 |
| OTU6 | 12 | 24 | 0.00006 | 5.68 | 0.0020 | -4,4 | 0,00325 | -12.8 | 0.0043 |
| OTU7 | 15 | 29 | 0.00005 | 4.96 | 0.0000 | -5,1 | 0,0004 | -12.9 | 0.0092 |
| OTU8 | 4 | 8 | 0.00055 | 2.84 | 0.1627 | -7,4 | 0,0510 | -27.0 | 0.0655 |
| OTU9 | 7 | 14 | 0.00005 | 11.14 | 0.0247 | -6,0 | 0,0056 | -15.4 | 0.0797 |
| OTU 10 | 4 | 8 | 0.00018 | 5.23 | 0.2216 | -5,1 | 0,0305 | -14.1 | 0.0301 |
| OTU11 | 6 | 12 | 0.00045 | 1.52 | 0.0122 | -4,2 | 0,0018 | -11.2 | 0.0737 |
| OTU 12 | 3 | 6 | 0.00035 | 4.50 | 0.2345 | -7,0 | 0,0916 | -22.4 | 0.2086 |
| OTU 13 | 5 | 10 | 0.00052 | 4.51 | 0.2760 | -6.1 | 0.0397 | -19.9 | 0.0127 |
| OTU 14 | 3 | 6 | 0.00025 | 10.04 | 0.3264 | -3.2 | 0.0147 | -9.7 | 0.0567 |
| OTU 15 | 2 | 4 | 0.00073 | 5.70 | 0.4820 | -4.2 | 0.0941 | -6.8 | 0.2463 |

De plus l'action précise de chacune des UTO 1 et UTO 2 a été évaluée en fonction des résultats de la population de l'étude. Les résultats sont présentés dans le Tableau 2 ci-après.

**Tableau 2**

| Bactéries | Résultats 16S Baseline Quantité de départ | évoluti on X fois | Δ% évolution du Poids | % vs. Evolution de la population totale | Δ% évolution de la masse grasse viscérale | % vs. Evolution MGV de la population totale |
|---|---|---|---|---|---|---|
| Population totale | 107 | | -3,6 | | -9,4 | |
| Population de l'étude sans les bactéries UTO 1 et UTO 2 | | | -3,4 | -6% | -8,9 | -5% |
| UTO 1 | 0,00043 | 5,9 | -4,8 | +33% | -12,8 | +36% |
| UTO 2 | 0.00013 | 3,0 | -4,8 | +33% | -15,4 | +64% |
| Total des 2 bactéries UTO 1 et UTO 2 | 0,00042 | 5,1 | -4,8 | +33% | -13,4 | +43% |

L'augmentation des bactéries selon l'invention dans le microbiome est directement liée à une diminution du poids et de la masse grasse chez l'homme. Les bactéries selon l'invention jouent donc un rôle primordial dans la lutte contre le surpoids, l'obésité, les maladies cardiométaboliques et les maladies inflammatoires, en particulier liés au gras viscéral, et peuvent être utilisées à cet effet.

Il a par ailleurs été constaté que les meilleurs résultats sont obtenus avec des personnes ayant un IMC supérieur à 30.

On constate également que les UTO 1 et 2 selon l'invention étaient déjà présentes en quantité suffisante pour être décelées et individualisées chez les patients de l'étude malgré leur surpoids ou obésité et le syndrome métabolique. La présence de ces bactéries dans un environnement hautement inflammatoire signifie qu'elles sont devenues résistantes à l'inflammation et que les deux gênes FHIT et TDRG1 ont partiellement résisté à l'épigénétique de l'alimentation ou de la transmission épigénétique des parents.

## Revendications

1. Bactérie commensale, héritable, gram négatif, strictement anaérobique et non sporulée de la famille des *Christensenellacées,* et appartenant à une UTO (Unité Taxonomique Opérationnelle) **caractérisée par** une séquence d'ARNr 16S SEQ ID N°1 pour une utilisation comme médicament.

2. Bactérie selon la revendication 1 pour une utilisation dans le traitement de l'obésité, des maladies cardiométaboliques et/ou des maladies inflammatoires de l'intestin.

3. Bactérie selon la revendication 1 pour une utilisation dans le traitement du syndrome métabolique, du pré-diabète, du diabète, des maladies vasculaires et cardiaques, de l'athérosclérose, de l'hyperlipidémie, de l'hyperglycémie, du NASH et/ou du NAFLD, de la maladie de Crohn.

4. Composition comprenant :
- au moins une bactérie commensale gram négatif, strictement anaérobique et non sporulée de la famille des *Christensenellacées,* et appartenant à une UTO **caractérisée par** une séquence d'ARNr 16S SEQ ID N°1, et
- au moins une bactérie commensale gram négatif, strictement anaérobique et non sporulée de la famille des *Christensenellacées,* et appartenant à une UTO **caractérisée par** une séquence d'ARNr 16S SEQ ID N°2.

5. Composition selon la revendication 4, **caractérisée en ce qu'**elle comprend également une bactérie commensale, héritable, de la famille des Christensenellacées et appartenant à une UTO **caractérisée par** une séquence d'ARNr 16S SEQ ID N°3 et/ou une bactérie appartenant à une UTO **caractérisée par** une séquence d'ARNr 16S SEQ ID N°4 et/ou une bactérie appartenant à une UTO **caractérisée par** une séquence d'ARNr 16S SEQ ID N°5 et/ou une bactérie appartenant à une UTO **caractérisée par** une séquence d'ARNr 16S SEQ ID N°6 et/ou une bactérie appartenant à une UTO **caractérisée par** une séquence d'ARNr 16S SEQ ID N°7 et/ou une bactérie appartenant à une UTO **caractérisée par** une séquence d'ARNr 16S SEQ ID N°8 et/ou une bactérie appartenant à une UTO **caractérisée par** une séquence d'ARNr 16S SEQ ID N°9 et/ou une bactérie appartenant à une UTO **caractérisée par** une séquence d'ARNr 16S SEQ ID N°10 et/ou une bactérie appartenant à une UTO **caractérisée par** une séquence d'ARNr 16S SEQ ID N°11 et/ou une bactérie appartenant à une UTO **caractérisée par** une séquence d'ARNr 16S SEQ ID N°12 et/ou une bactérie appartenant à une UTO **caractérisée par** une séquence d'ARNr 16S SEQ ID N°13 et/ou une bactérie appartenant à une UTO **caractérisée par** une séquence d'ARNr 16S SEQ ID N°14 et/ou une bactérie appartenant à une UTO **caractérisée par** une séquence d'ARNr 16S SEQ ID N°15.

6. Composition selon l'une des revendications 4 à 5, **caractérisée en ce qu'**elle comprend également une ou plusieurs bactéries méthanogènes de la famille des *Methanobacteriaceae,* du genre *Methanobacterium* et/ou *Methanobrevibacter* et/ou *Methanosphaera* et/ou *Methanothermobacter,* et/ou la bactérie *Methanobrevibacter smithii.*

7. Composition selon l'une des revendications 4 à 5, **caractérisée en ce qu'**elle comprend également une ou plusieurs bactéries des espèces, genres ou ordres suivants : *Marvinbryantia formatexigens* et/ou *Bacteroides thetaiotaomicron* et/ou *Akkermansia muciniphila* et/ou *Faecalibacterium prausnitzii* et/ou *Clostridium thermocellum* et/ou *Dehalobacteriaceae et*/*ou Oscillospira et*/*ou Mogibacteriaceae et*/*ou Ruminococcaceae et*/*ou Ruminococcus et*/*ou Lachinospiraceae et*/*ou Lachinospira et*/*ou Bacteroidaceae et*/*ou Rikenellaceae et*/*ou Clostridium et*/*ou Clostridiales.*

8. Composition selon l'une des revendications 4 à 7, **caractérisée en ce qu'**elle comprend également au moins une autre bactérie choisie parmi *Christensenella massiliensis, Christensenella timonensis* et *Christensenella minuta.*

9. Composition la revendication 4 à 8, **caractérisée en ce que** les bactéries sont présentes sous forme lyophilisée.

10. Composition selon l'une des revendications 4 à 9, **caractérisée en ce que** les bactéries présentes sont pour au moins 50% des bactéries vivantes (en nombre).

11. Composition selon l'une des revendications 4 à 10, **caractérisée en ce que** les bactéries présentes sont pour au moins 90% des bactéries vivantes (en nombre).

12. Composition selon l'une des revendications 4 à 11, **caractérisée en ce qu'**elle comprend également au moins un acide aminé et/ou au moins un peptide.

13. Composition selon l'une des revendications 4 à 12, **caractérisée en ce qu'**elle comprend également au moins un acide aminé choisi parmi la thréonine, la leucine, la sérine, la proline, l'alanine, la glycine, la glutamine, l'acide glutamique, le tryptophane et/ou au moins un peptide contenant au moins un de ces acides aminés.

14. Composition selon l'une des revendications 4 à 13, **caractérisée en ce qu'**elle comprend au moins un probiotique et/ou au moins un prébiotique.

15. Composition selon l'une des revendications 4 à 14, **caractérisée en ce qu'**elle se présente sous forme de poudre, de poudre microencapsulée, de gélule, de capsugel, de comprimé, ou de transplant fécal.

16. Composition selon l'une des revendications 4 à 15, **caractérisée en ce qu'**elle se présente sous une forme gastro-résistante.

17. Composition selon l'une des revendications 4 à 16, pour une utilisation comme médicament.

## Patentansprüche

1. Kommensales, vererbbares, gramnegatives, streng anaerobes und nicht sporuliertes Bakterium aus der Familie der *Christensenellaceae,* das zu einer UTO (Operational Taxonomic Unit) gehört, die durch eine rRNA-Sequenz 16S SEQ ID Nr. 1 gekennzeichnet ist, zur Verwendung als Arzneimittel.

2. Bakterium nach Anspruch 1 zur Verwendung bei der Behandlung von Fettleibigkeit, kardiometabolischen Erkrankungen und/oder entzündlichen Darmerkrankungen.

3. Bakterium nach Anspruch 1 zur Verwendung bei der Behandlung von metabolischem Syndrom, Prädiabetes, Diabetes, Gefäß- und Herzerkrankungen, Atherosklerose, Hyperlipidämie, Hyperglykämie, NASH und/oder NAFLD, Morbus Crohn.

4. Zusammensetzung, umfassend:
- mindestens ein kommensales, gramnegatives, streng anaerobes und nicht sporuliertes Bakterium aus der Familie der *Christensenellaceae,* das zu einer UTO gehört, die durch eine rRNA-Sequenz 16S SEQ ID Nr. 1 gekennzeichnet ist, und
- mindestens ein kommensales, gramnegatives, streng anaerobes und nicht sporuliertes Bakterium aus der Familie der *Christensenellaceae,* das zu einer UTO gehört, die durch eine rRNA-Sequenz 16S SEQ ID Nr. 2 gekennzeichnet ist.

5. Zusammensetzung nach Anspruch 4, **dadurch gekennzeichnet, dass** sie ebenfalls ein kommensales, vererbbares Bakterium aus der Familie der Christensenellaceae umfasst, das zu einer UTO gehört, die durch eine RNA-Sequenz 16 S SEQ ID Nr. 3 gekennzeichnet ist und/oder ein Bakterium, das zu einer UTO gehört, die durch eine rRNA-Sequenz 16S SEQ ID Nr. 4 gekennzeichnet ist und/oder ein Bakterium, das zu einer UTO gehört, die durch eine rRNA-Sequenz 16S SEQ ID Nr. 5 gekennzeichnet ist und/oder ein Bakterium, das zu einer UTO gehört, die durch eine rRNA-Sequenz 16S SEQ-ID Nr. 6 gekennzeichnet ist und/oder ein Bakterium, das zu einer UTO gehört, die durch eine rRNA-Sequenz 16S SEQ ID Nr. 7 gekennzeichnet ist, und/oder ein Bakterium, das zu einer UTO gehört, die durch eine rRNA-Sequenz 16S SEQ-ID Nr. 8 gekennzeichnet ist und/oder ein Bakterium, das zu einer UTO gehört, die durch eine rRNA-Sequenz 16 S SEQ ID Nr. 9 gekennzeichnet ist und/oder ein Bakterium, das zu einer UTO gehört, die durch eine rRNA-Sequenz 16S SEQ ID Nr. 10 gekennzeichnet ist und/oder ein Bakterium, das zu einer UTO gehört, die durch eine rRNA-Sequenz 16S SEQ ID Nr. 11 gekennzeichnet ist und/oder ein Bakterium, das zu einer UTO gehört, die durch eine rRNA-Sequenz 16S SEQ ID Nr. 12 gekennzeichnet ist und/oder ein Bakterium, das zu einer UTO gehört, die durch eine rRNA-Sequenz 16S SEQ ID Nr. 13 gekennzeichnet ist und/oder ein Bakterium, das zu einer UTO gehört, die durch eine rRNA-Sequenz 16S SEQ ID Nr. 14 gekennzeichnet ist und/oder ein Bakterium, das zu einer UTO gehört, die durch eine rRNA-Sequenz 16S SEQ ID Nr. 15 gekennzeichnet ist.

6. Zusammensetzung nach einem der Ansprüche 4 bis 5, **dadurch gekennzeichnet, dass** sie ebenfalls ein oder mehrere methanogene Bakterien aus der Familie der *Methanobacteriaceae* der Gattung *Methanobacterium* und/oder *Methanobrevibacter* und/oder *Methanosphaera* und/oder *Methanothermobacter* und/oder das Bakterium *Methanobrevibacter smithii* umfasst.

7. Zusammensetzung nach einem der Ansprüche 4 bis 5, **dadurch gekennzeichnet, dass** sie ebenfalls eine oder mehrere Bakterien der folgenden Arten, Gattungen oder Ordnungen umfasst:
*Marvinbryantia formatexigens* und/oder *Bacteroides thetaiotaomicron* und/oder *Akkermansia muciniphila* und/oder *Faecalibacterium prausnitzii* und/oder *Clostridium thermocellum* und/oder *Dehalobacteriaceae* und/oder *Oscillospira* und/oder *Mogibacteriaceae* und/oder *Ruminococcaceae* und/oder *Ruminococcus* und/oder *Lachinospiraceae* und/oder *Lachinospira* und/oder *Bacteroidaceae* und/oder *Rikenellaceae* und/oder *Clostridium* und/oder *Clostridiales.*

8. Zusammensetzung nach einem der Ansprüche 4 bis 7, **dadurch gekennzeichnet, dass** sie ebenfalls mindestens ein anderes Bakterium umfasst, das aus *Christensenella massiliensis, Christensenella timonensis* und *Christensenella minuta* ausgewählt ist.

9. Zusammensetzung nach Anspruch 4 bis 8, **dadurch gekennzeichnet, dass** die Bakterien in lyophilisierter Form vorliegen.

10. Zusammensetzung nach einem der Ansprüche 4 bis 9, **dadurch gekennzeichnet, dass** die vorhandenen Bakterien (der Anzahl nach) zu mindestens 50 % lebende Bakterien sind.

11. Zusammensetzung nach einem der Ansprüche 4 bis 10, **gekennzeichnet dadurch, dass** die vorhandenen Bakterien (der Anzahl nach) zu mindestens 90 % lebende Bakterien sind.

12. Zusammensetzung nach einem der Ansprüche 4 bis 11, **dadurch gekennzeichnet, dass** sie ebenfalls mindestens eine Aminosäure und/oder mindestens ein Peptid umfasst.

13. Zusammensetzung nach einem der Ansprüche 4 bis 12, **dadurch gekennzeichnet, dass** sie ebenfalls mindestens eine Aminosäure umfasst, die aus Threonin, Leucin, Serin, Prolin, Alanin, Glycin, Glutamin, Glutaminsäure, Tryptophan ausgewählt ist und/oder mindestens ein Peptid, das mindestens eine dieser Aminosäuren enthält.

14. Zusammensetzung nach einem der Ansprüche 4 bis 13, **dadurch gekennzeichnet, dass** sie mindestens ein Probiotikum und/oder mindestens ein Präbiotikum umfasst.

15. Zusammensetzung nach einem der Ansprüche 4 bis 14, **dadurch gekennzeichnet, dass** sie in Form von Pulver, mikroverkapseltem Pulver, Weichkapsel, Capsugel, Tablette oder Fäkaltransplantat vorliegt.

16. Zusammensetzung nach einem der Ansprüche 4 bis 15, **dadurch gekennzeichnet, dass** sie in gastro-resistenter Form vorliegt.

17. Zusammensetzung nach einem der Ansprüche 4 bis 16 zur Verwendung als Arzneimittel.

## Claims

1. Commensal, heritable, gram-negative, strictly anaerobic and non-sporulated bacterium of the *Christensenellaceae* family, belonging to an OTU (Operational Taxonomic Unit) **characterized by** a 16S rRNA sequence SEQ ID NO: 1 for use as a drug.

2. Bacterium according to claim 1 for use in the treatment of obesity, cardiometabolic diseases and/or inflammatory bowel diseases.

3. Bacterium according to claim 1 for use in the treatment of metabolic syndrome, pre-diabetes, diabetes, vascular and heart diseases, atherosclerosis, hyperlipidemia, hyperglycemia, NASH and/or NAFLD, and Crohn's disease.

4. Composition comprising:
- at least one commensal, gram-negative, strictly anaerobic and non-sporulated bacterium of the *Christensenellaceae* family, belonging to an OTU **characterized by** a 16S rRNA sequence SEQ ID NO: 1, and
- at least one commensal, gram-negative, strictly anaerobic and non-sporulated bacterium of the *Christensenellaceae* family, belonging to an OTU **characterized by** a 16S rRNA sequence SEQ ID NO: 2.

5. Composition according to claim 4, **characterized in that** it also comprises a commensal, heritable bacterium of the Christensenellaceae family, belonging to an OTU **characterized by** a 16S rRNA sequence SEQ ID NO: 3 and/or a bacterium belonging to an OTU **characterized by** a 16S rRNA sequence SEQ ID NO: 4 and/or a bacterium belonging to an OTU **characterized by** a 16S rRNA sequence SEQ ID NO: 5 and/or a bacterium belonging to an OTU **characterized by** a 16S rRNA sequence SEQ ID NO: 6 and/or a bacterium belonging to an OTU **characterized by** a 16S rRNA sequence SEQ ID NO: 7 and/or a bacterium belonging to an OTU **characterized by** a 16S rRNA sequence SEQ ID NO: 8 and/or a bacterium belonging to an OTU **characterized by** a 16S rRNA sequence SEQ ID NO: 9 and/or a bacterium belonging to an OTU **characterized by** a 16S rRNA sequence SEQ ID NO: 10 and/or a bacterium belonging to an OTU **characterized by** a 16S rRNA sequence SEQ ID NO: 11 and/or a bacterium belonging to an OTU **characterized by** a 16S rRNA sequence SEQ ID NO: 12 and/or a bacterium belonging to an OTU **characterized by** a 16S rRNA sequence SEQ ID NO: 13 and/or a bacterium belonging to an OTU **characterized by** a 16S rRNA sequence SEQ ID NO: 14 and/or a bacterium belonging to an OTU **characterized by** a 16S rRNA sequence SEQ ID NO: 15.

6. Composition according to either claim 4 or claim 5, **characterized in that** it also comprises one or more methanogenic bacteria of the *Methanobacteriaceae* family, of the genus *Methanobacterium* and/or *Methanobrevibacter* and/or *Methanosphaera* and/or *Methanothermobacter,* and/or the *Methanobrevibacter smithii* bacterium.

7. Composition according to either claim 4 or claim 5, **characterized in that** it also comprises one or more bacteria of the following species, genera or orders:
*Marvinbryantia formatexigens* and/or *Bacteroides thetaiotaomicron* and/or *Akkermansia muciniphila* and/or *Faecalibacterium prausnitzii* and/or *Clostridium thermocellum* and/or *Dehalobacteriaceae* and/or *Oscillospira* and/or *Mogibacteriaceae* and/or *Ruminococcaceae* and/or *Ruminococcus* and/or *Lachinospiraceae* and/or *Lachinospira* and/or *Bacteroidaceae* and/or *Rikenellaceae* and/or *Clostridium* and/or *Clostridiales.*

8. Composition according to any of claims 4 to 7, **characterized in that** it also comprises at least one other bacterium selected from *Christensenella massiliensis, Christensenella timonensis* and *Christensenella minuta.*

9. Composition according to any of claims 4 to 8, **characterized in that** the bacteria are present in freeze-dried form.

10. Composition according to any of claims 4 to 9, **characterized in that** the bacteria present are at least 50% living bacteria (by number).

11. Composition according to any of claims 4 to 10, **characterized in that** the bacteria present are at least 90% living bacteria (by number).

12. Composition according to any of claims 4 to 11, **characterized in that** it also comprises at least one amino acid and/or at least one peptide.

13. Composition according to any of claims 4 to 12, **characterized in that** it also comprises at least one amino acid selected from threonine, leucine, serine, proline, alanine, glycine, glutamine, glutamic acid, tryptophan and/or at least one peptide containing at least one of these amino acids.

14. Composition according to any of claims 4 to 13, **characterized in that** it comprises at least one probiotic and/or at least one prebiotic.

15. Composition according to any of claims 4 to 14, **characterized in that** it is provided in powder, micro-encapsulated powder, capsule, capsugel, tablet or fecal transplant form.

16. Composition according to any of claims 4 to 15, **characterized in that** it is provided in a gastro-resistant form.

17. Composition according to any of claims 4 to 16, for use as a drug.


### SEQUENCE LISTING

<110> International Nutrition Research Company
<120> BACTERIES SPECIFIQUES POUR LEUR UTILISATION COMME MEDICAMENT
   EN PARTICULIER POUR LUTTER CONTRE LE SURPOIDS, LOBESITE, LES MALADIES
   CARDIOMETABOLIQUES ET LES MALADIES INFLAMMATOIRES DE LINTESTIN
<130> LabNut.011
<160> 15
<170> BiSSAP 1.3.6
<210> 1
   <211> 407
   <212> RNA
   <213> Christensenellaceae
<220>
   <223> OTU 1 16S rRNA
<400> 1
   tggggaatat tgggcaatgg gcgaaagcct tacccagcaa cgccgcgtga gggaagaagg      60
   ttttcggatt gtaaacctct gtcctggggg acgaaggaag tgacggtacc ccgggaggaa     120
   gccccggcta actacgtgcc agcagccgcg gtaatacgta gggggcgagc gttgtccgga     180
   attattgggc gtaaagggcg cgtagacggc tgtgtaagtt ataggtgaaa gcccaacttt     240
   caaggttgga actgcctgta atactgcatg gcttgagtgc aggagaggga agcggaattc     300
   ctagtgtagc ggtgaaatgc gtagatatta ggaggaacac cagtggcgaa ggcggctttc     360
   tggactgtaa ctgacgttga ggcgcgaaag cgtggggagc aaacagg                   407
<210> 2
   <211> 407
   <212> RNA
   <213> Christensenellaceae
<220>
   <223> OTU 2 16S rRNA
<400> 2
   gggggaatat tgggcaatgg gggaaaccct gacccagcaa cgccgcgtga aggaagaagg      60
   ccttcgggtt gtaaacttct gtgacagggg acgaaggaag tgacggtacc ctgagaggaa     120
   gctccggcaa actacgtgcc agcagccgcg gtaatacgta gggagcgagc gttgtccgga     180
   attactgggc gtaaagggcg cgtaggcggt agatcaagtc gcatgtgaaa tacccgggct     240
   caacctgggg ggtgcatgcg aaactggaat acttgagtgc ggtagaggaa agtggaattc     300
   ctagtgtagc ggtgaaatgc gtagatatta ggaggaacac cagtggcgaa ggcgactttc     360
   tgggccgtaa ctgacgctga ggcgcgaaag cgtggggagc aaacagg                   407
<210> 3
   <211> 407
   <212> RNA
   <213> Christensenellaceae
<220>
   <223> OTU 3 16S rRNA
<400> 3
   tggggaatat tgggcaatgg gcgcaagcct gacccagcaa cgccgcgtga gggaagaagg 60
   ttttcggatt gtaaacctct gtcgcagaag acgaaggaag tgacggtatt ctgtgaggaa 120
   gccccggcta actacgtgcc agcagccgcg gtaatacgta gggggcgagc gttgtccgga 180
   attactgggc gtaaagggag cgtaggcggt ctgataagtt ggatgtgaaa tacccgggct 240
   taacttgggg ggtgcatcca atactgttgg actagagtac aggagaggaa agcggaattc 300
   ctagtgtagc ggtgaaatgc atagatatta ggaggaacat cggtggcgaa ggcggctttc 360
   tggactgcaa ctgacgctga ggctcgaaag cgtggggagc aaacagg 407
<210> 4
   <211> 407
   <212> RNA
   <213> Christensenellaceae
<220>
   <223> OTU 4 16S rRNA
<400> 4
   tagggaatat tggacaatgg ggggaaccct gatccagcaa cgccgcgtga aggaagaagg 60
   tcttcggatt gtaaacttat gttgtcgggg acgaagaaag acggtacccg acgaggaagc 120
   cccggctaac tacgtgccag cagccgcggt aatacgtagg gggcgagcgt tgtccggaat 180
   tactgggcgt aaagggcgcg taggcggcat attaagttag atgtgaaatc cccgggctta 240
   acctgggtgt tgcatttaaa actgataagc ttgagtgcag gagagggaag cggaattcct 300
   agtgtagcgg tggaatgcgt agatattagg aggaacacca gtggcgaagg cggctttctg 360
   gactgtaact gacgctgagg cgcgagagcg tggggagcaa acaggcc 407
<210> 5
   <211> 407
   <212> RNA
   <213> Christensenellaceae
<220>
   <223> OTU 5 16S rRNA
<400> 5
   tagggaatat tgggcaatgg gcgcaagcct gacccagcaa cgccgcgtga gggaagaagg 60
   ttttcggatt gtaaacctct gtcctatgtg acgaaggaag tgacggtagc ataggaggaa      120
   gccccggcta actacgtgcc agcagccgcg gtaatacgta gggggcgagc gttgtccgga      180
   attactgggc gtaaagggtg cgtaggcggt ttggtaagtt ggatgtgaaa tacccgggct      240
   taacttgggg gctgcatcca atactgtcgg acttgagtgc aggagaggaa agcggaattc      300
   ctagtgtagc ggtgaaatgc gtagatatta ggaggaacac cagtggcgaa ggcggcttgc      360
   tggacagtaa ctgacgctga ggcgcgaaag cgtggggagc aaacagg                    407
<210> 6
   <211> 407
   <212> RNA
   <213> Christensenellaceae
<220>
   <223> OTU 6 16S rRNA
<400> 6
   tagggaatat tgggcaatgg gcgcaaccct gacccagcaa cgccgcgtga gggaagaagg       60
   ttctctggat tgtaaacctc tgtcctatgg gacgaagaag tgacggtacc ataggaggaa      120
   gctccggcta actacgtgcc agcagccgcg gtaatacgta gggagcgagc gttgtccgga      180
   attactgggc gtaaagggtg cgtaggcggc ctggtaagtc agatgtgaaa tacccgtgct      240
   caacatgggg ggtgcatctg atactgttag gcttgagtgc aggagaggaa agcggaattc      300
   ctagtgtagc ggtgaaatgc gtagatatta ggaggaacac cagtggcgaa ggcggctttc      360
   tggactgtaa ctgacgctga ggcacgaaag cgtggggagc aaacagg                    407
<210> 7
   <211> 407
   <212> RNA
   <213> Christensenellaceae
<220>
   <223> OTU 7 16S rRNA
<400> 7
   tggggaatat tgggcaatgg gggcaaccct gacccagcaa cgccgcgtga gggaagaagg 60
   ttttcggatt gtaaacctct gtccttgggg acgaagaagt gacggtaccc aaggaggaag 120
   ctccggctaa ctacgtgcca gcagccgcgg taatacgtag ggagcgagcg ttgtccggaa 180
   ttactgggcg taaagggtgc gtaggcggtt tggtaagtca gatgtgaaat acccgggctt 240
   aacccggggg ctgcatctga tactgtcaga cttgagtgca ggagaggaaa gcggaattcc 300
   tagtgtagcg gtgaaatgcg tagatattag gaggaacacc agtggcgaag gcggctttct 360
   ggactgtaac tgacgctgag gcacgaaagc gtggggagca aacaggg 407
<210> 8
   <211> 407
   <212> RNA
   <213> Christensenellaceae
<220>
   <223> OTU 8 16S rRNA
<400> 8
   tcgggaatat tggacaatgg gggaaaccct gatccagcaa cgccgcgtga aggaagaagg 60
   tcttcggatt gtaaacttat gttgtcgggg acgaagaaag acggtacccg acgaggaagc 120
   cccggctaac tacgtgccag cagccgcggt aatacgtagg gggcgagcgt tgtccggaat 180
   tactgggcgt aaagggcgcg taggcggcca attaagttag atgtgaaatc cccgggctta 240
   acctgggtgt tgcatttaaa actgataggc ttgagtgcag gagagggaag cggaattcct 300
   agtgtagcgg tggaatgcgt agatattagg aggaacacca gtggcgaagg cggctttctg 360
   gactgtaact gacgctgagg cgcgagagcg tggggagcaa acaggcc 407
<210> 9
   <211> 407
   <212> RNA
   <213> Christensenellaceae
<220>
   <223> OTU 9 16S rRNA
<400> 9
   tggggaatat tgggcaatgg gcggaagcct gacccagcaa cgccgcgtga gggaagaagg      60
   ttttcggatt gtaaacctct gtcgcagaag acgaaggaag tgacggtatt ctgtgaggaa     120
   gccccggcta actacgtgcc agcagccgcg gtaatacgta gggggcgagc gttgtccgga     180
   attactgggc gtaaagggtg cgtaggcggt tgcttaagtt ggatgtgaaa tacccgggct     240
   taacttgggg ggtgcattca agactgggga actagagtac aggagaggga agcggaattc     300
   ctagtgtagc ggtgaaatgc gtagatatta ggaggaacac cggtggcgaa ggcggctttc     360
   tggactgaca ctgacgctga ggcacgaaag cgtggggagc aaacagg                   407
<210> 10
   <211> 407
   <212> RNA
   <213> Christensenellaceae
<220>
   <223> OTU 10 16S rRNA
<400> 10
   tggggaatat tgggcaatgg gcgcaagcct gacccagcaa cgccgcgtga gggaagaagg      60
   ttttcggatc gtaaacctct gttgcagggg acgaggagta gacggtaccc tacgaggaag     120
   ctccggctaa ctacgtgcca gcagccgcgg taatacgtag ggagcaagcg ttgtccggaa     180
   tgactgggcg taaagggcgc gtaggtggtt ttgtaagtct ggagtgaaac ccccgttttc      240
   aaggcggggc ctgctttgga gactgcagga cttgagtgat ggagaggata gcggaattcc     300
   cggtgtagcg gtgaaatgcg tagagatcgg gaggaacacc agtggcgaag gcggctatct     360
   ggacatagac tgacactgag gcgcgaaagc gtggggagca aacaggc                   407
<210> 11
   <211> 407
   <212> RNA
   <213> Christensenellaceae
<220>
   <223> OTU 11 16S rRNA
<400> 11
   tggggaatat tgggcaatgg gcgcaagcct gacccagcaa cgccgcgtga gggaagaagg      60
   tcttcggatt gtaaacctct gtcgcaaggg acgaaggcgt gacggtacct tgtgaggaag     120
   ccccggctaa ctacgtgcca gcagccgcgg taatacgtag ggggcgagcg ttgtccggaa     180
   ttactgggcg taaagggtgc gtaggcggtt gcttaagttg gatgtgaaat acccgggctt     240
   aacttggggg gtgcattcaa aactgggcga ctagagttca ggagagggaa gcggaattcc     300
   tagtgtagcg gtgaaatgcg tagatattag gaggaacacc agtggcgaag gcggctttct     360
   ggactataac tgacgctgag gcacgaaagc gtggggagca aacaggc                   407
<210> 12
   <211> 407
   <212> RNA
   <213> Christensenellaceae
<220>
   <223> OTU 12 16S rRNA
<400> 12
   tggggaatat tgggcaatgg gcgcaaccct gacccagcaa cgccgcgtga gggaagaagg      60
   ttttcggatt gtaaacctct gtcgcagaag acgaaggaag tgacggtatt ctgtgaggaa     120
   gccccggcta actacgtgcc agcagccgcg gtaatacgta gggggcgagc gttgtccgga     180
   attactgggc gtaaagggtg cgtaggcggt taattaagtt ggatgtgaaa ttcccgggct     240
   taacttggga gctgcattca aaactggtta actagagttc aggagaggga agcggaattc     300
   ctagtgtagc ggtgaaatgc gtagatatta ggaggaacac cagtggcgaa ggcgacttac     360
   tggacgaaca ctgacgctga ggcacgaaag cgtggggagc aaacagg                   407
<210> 13
   <211> 407
   <212> RNA
   <213> Christensenellaceae
<220>
   <223> OTU 13 16S rRNA
<400> 13
   tggggaatat tgggcaatgg gcgcaagcct gacccagcaa cgccgcgtga aggaagaagg 60
   tcttcggatt gtaaactttt gtcgccggga acgataatga cggtacccgg taagaaagcc 120
   ccggctaact acgtgccagc agccgcggta atacgtaggg ggcgagcgtt gtccggaatt 180
   actgggcgta aagggcgcgt aggcggccgg ataagttaga tgtgaaatcc ccgggctcaa 240
   cctgggtgtt gcatttaaaa ctgtctggct tgagtgcagg agaggaaagc ggaattccta 300
   gtgtagcggt gaaatgcgta gatattagga ggaacatcag tggcgaaggc ggctttctgg 360
   actgtaactg acgctgaggc gcgaaagcgt ggggagcaaa caggccc 407
<210> 14
   <211> 407
   <212> RNA
   <213> Christensenellaceae
<220>
   <223> OTU 14 16S rRNA
<400> 14
   tggggaatat tgggcaatgg gcgaaagcct gacccagcaa cgccgcgtga gggaagaagg 60
   ccttcgggtt gtaaacctct gtccttagtg acgaaggaag tgacggtagc taaggaggaa 120
   gccccggcta actacgtgcc agcagccgcg gtaatacgta gggggcgagc gttgtccgga 180
   attactgggc gtaaagggtg cgtaggcggt actttaagtt ggatgtgaaa tccccgggct 240
   taacctgggg cctgcatcca atactggaga actagagtgc aggagaggga agcggaattc 300
   ctagtgtagc ggtgaaatgc gtagatatta ggaagaacac cagtggcgaa ggcggctttc 360
   tggactgtaa ctgacgctga ggcacgaaag cgtggggagc aaacagg 407
<210> 15
   <211> 407
   <212> RNA
   <213> Christensenellaceae
<220>
   <223> OTU 15 16S rRNA
<400> 15
   tggggaatat tgcgcaatgg gcgcaagcct gacgcagcaa cgccgcgtga gggaagaagg 60
   ctttcgggtt gtaaacctct gtccttggtg acgaaggaag tgacggtagc caaggaggaa 120
   gccccggcta actacgtgcc agcagccgcg gtaatacgta gggggcgagc gttgtccgga     180
   attactgggc gtaaagggtg cgtaggcggt ctattaagtt ggatgtgaaa tccccgggct     240
   taacttgggg tctgcatcca atactgggag actagagtgc aggagaggga agcggaattc     300
   ctagtgtagc ggtgaaatgc gtagatatta ggaggaacac cagtggcgaa ggcggctttc     360
   tggactgtaa ctgacgctga ggcacgaaag cgtggggagc aaacagg                   407